# EUROPEAN PATENT APPLICATION

(11) **EP 2 930 171 A1**
(43) Date of publication of application: **14.10.2015**
(21) Application number: 14163774.4
(22) Date of filing: 07.04.2014
(51) Int. Cl.: C07D 295/096, A61K 31/495, A61P 25/24

(54) **Synthesis of vortioxetine via (2,4-dimethylphenyl)(2-iodophenyl)sulfane intermediate**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention provides a new synthetic process for the production of 1-(2-((2,4-dimethylphenyl)thio)phenyl)piperazine (vortioxetine), a drug for the treatment of depression and anxiety, which is conducted via (2,4-dimethylphenyl)(2-iodophenyl)sulfane intermediate.

## Description

### Field of the Invention

This invention relates to a new and advantageous process for the synthesis of 1-(2-((2,4-dimethylphenyl)thio)phenyl)piperazine (vortioxetine) a drug for the treatment of depression and anxiety.

### Background of the Invention

Vortioxetine is disclosed as Example 1e in WO 2003/029232 A1 and is described as being prepared analogously to Example 1. The process used to prepare Example 1 involves the preparation of 1-(2-((2-(trifluoromethyl)phenyl)thio)phenyl)piperazine on a solid polystyrene support, followed by decomplexation using visible light irradiation, and purification by preparative LC-MS and ion-exchange chromatography. The overall yield for the preparation of vortioxetine is described as 17%.

Several alternative palladium catalyzed processes for the preparation of vortioxetine are described in Examples 17 to 25 of WO 2007/144005 A1. These processes describe the preparation of vortioxetine from 2,4-dimethylthiophenol and 2-bromoiodobenzene (or 1,2-dibromobenzene) starting materials *via* a 1-(2-bromo-phenylsulfanyl)-2,4-dimethyl-benzene intermediate. Each of these processes involves the use of a palladium catalyst and a phosphine ligand.

The preparation of vortioxetine is also described by Bang-Andersen et al. in J. Med. Chem. (2011), Vol. 54, 3206-3221. Here, in a first step, tert-butyl 4-(2-bromophenyl)piperazine-1-carboxylate intermediate is prepared from Boc-piperazine and 2-bromoiodobenzene in a palladium catalyzed coupling reaction. *tert*-Butyl 4-(2-bromophenyl)piperazine-1-carboxylate is then reacted with 2,4-dimethylthiophenol, again in the presence of palladium catalyst and a phosphine ligand, to provide Boc-protected vortioxetine. In the final step, vortioxetine is deprotected using hydrochloric acid to give vortioxetine hydrochloride.

WO 2013/102573 A1 describes a reaction between 1-halogen-2,4-dimethyl-phenyl, 2-halogenthiophenol and an optionally protected piperazine in the presence of a base and a palladium catalyst consisting of a palladium source and a phosphine ligand.

Each of the above processes has disadvantages. The process described in WO 2003/029232 is low yielding and unsuitable for the large scale production of vortioxetine, whereas the processes described in WO 2007/144005 A1, WO 2013/102573 A1 and by Bang-Andersen *et al.* require the use of expensive starting materials, palladium catalyst and phosphine ligand. In addition, the toxicity of palladium is well known, Liu et al. Toxicity of Palladium, Toxicology Letters, 4 (1979) 469-473, and the European Medicines Agency' s Guideline on the Specification for Residues of Metal Catalysts sets clear limits on the permitted daily exposure to palladium arising from palladium residue within drug substances, www.ema.europa.eu. Thus it would be desirable to avoid the use of a palladium catalyst in the synthesis of vortioxetine and the subsequent purification steps required to remove palladium residue from the final pharmaceutical product.

### Summary of the Invention

Various aspects, advantageous features and preferred embodiments of the present invention as summarized in the following items, respectively alone or in combination, contribute to solving the object of the invention.
(1) A process for the preparation of a compound of formula **VII,** or a salt thereof, 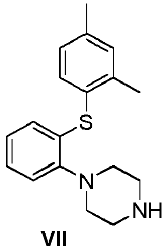 said process comprising the steps of
   a) reacting a compound of formula V 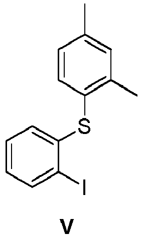 with piperazine in a presence of a copper catalyst, and
   b) optionally, converting the obtained compound of formula **VII** into the salt thereof.
(2) The process according to item 1, wherein a Cu(I) salt is used as the copper catalyst.
(3) The process according to item 2, wherein the Cu(I) salt is selected from the group consisting of CuI, CuBr, CuCl and Cu₂O.
(4) The process according to item 2 or item 3, wherein the copper catalyst additionally comprises a ligand.
(5) The process according to item 4, wherein the ligand is selected from the group consisting of amino acids, bipyridines, diamines, phenols, alcohols, 1H-benzotriazole, 2-((2,6-dimethylphenyl)amino)-2-oxoacetic acid, and 2-isobutyrylcyclohexanone.
(6) The process according to item 5, wherein the ligand is 2-phenylphenol or N,N-diethyl-2-hydroxybenzamide.
(7) The process according to item 1, wherein in step a) a base is present.

The term "base" as employed herein means a proton acceptor, preferably a water soluble proton acceptor or an alcoholate salt such as an alkali metal *tert*-butanolate salt, more preferably the proton acceptor is selected from the group consisting of carbonate salts, and hydroxides of alkaline or earth alkaline metals. The preferred bases are K₃PO₄, Cs₂CO₃ and K₂CO₃.
(8) The process according to item 7, wherein the base is K₃PO₄.
(9) The process according to item 1, wherein step a) is conducted at a temperature in a range from 20°C to 200°C.
(10) The process according to item 9, wherein the temperature is about 120°C.

The term "about" means approximately, in the region of, roughly, or around. When the term is used in conjunction with a numerical range, it modifies that range by extending the boundaries above and below the numerical values set forth. In general, the term "about" is used herein to modify a numerical value above and below the stated value by a variance of 10 %.
(11) The process according to item 1, wherein the compound of formula **V** is prepared by reacting a compound of formula **I** 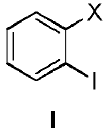 wherein X represents F or NH₂,
   with a compound of formula **II** 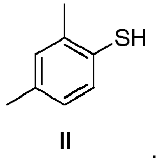
(12) The process according to item 1, wherein the compound of formula V is prepared by reacting a compound of formula I' 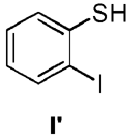 with a compound of formula **II'** 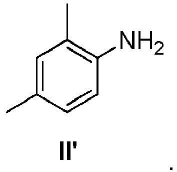
(13) The process according to item 12, wherein said process is conducted in a presence of a copper catalyst.
(14) The process according to item 1, wherein the compound of formula V is prepared by
   reacting a compound of formula **III** 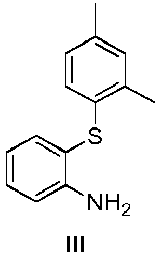 with nitrite, and
   reacting the obtained diazo compound of formula **IV** 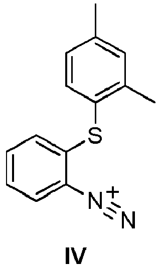 with a compound of formula **MI,** wherein M is selected from the group consisting of K, Na, Li, Cs, Zn, Cu, NH₄, and Bu₄N, or with a compound of formula **NI₂,** wherein N is selected from the group consisting of Mg, Ca, Ba, Fe, Cu, Ni, and Sn, or with AlI₃.
(15) The process according to item 14, wherein the obtained diazo compound of formula **IV** is reacted with a compound of formula **MI,** wherein M is selected from the group consisting of K, Na, Li, Cs, Zn, Cu, NH₄, and Bu₄N, preferably M is K.
(16) The process according to item 14, wherein the obtained diazo compound of formula **IV** is reacted with a compound of formula **NI₂,** wherein N is selected from the group consisting of Mg, Ca, Ba, Fe, Cu, Ni, and Sn.
(17) The process according to item 14, wherein the compound of formula **III** is prepared by reacting a compound of formula **I"** 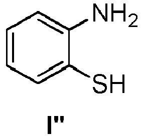 with a compound of formula **II"** 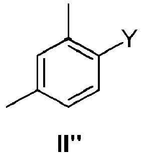 wherein Y represents Br or I.
(18) The process according to item 14, wherein the compound of formula **III** is prepared by
   a) reacting a compound of formula **I"'** 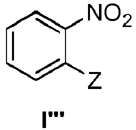 wherein Z represents F or Cl, with a compound of formula **II** 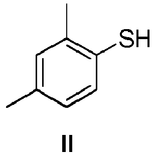 to obtain the compound of formula **III'** 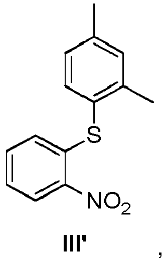 and
   b) reducing the compound of formula **III'** to obtain the compound of formula **III.**
(19) The process according to any one of the previous items, wherein the salt of the compound of formula **VII** is a hydrobromide salt or a hydrochloride salt.
(20) A compound of formula V 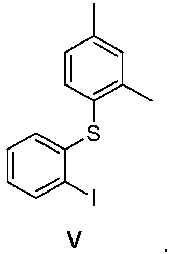
(21) Use of the compound as defined in item 20 in the preparation of vortioxetine or a salt thereof.
(22) A process for the preparation of a pharmaceutical composition comprising a compound of
   formula **VII,** or a salt thereof 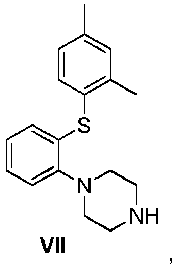 comprising the steps of:
   x) preparing a compound of formula **VII,** or a salt thereof, by carrying out a process according to any one of items 1 to 19, and
   y) mixing the compound of formula **VII,** or a salt thereof, with a pharmaceutically acceptable carrier and/or excipient.

The new process defined above provides vortioxetine, or salts thereof, in a high yield without the need of using the homogeneous catalysis with palladium catalysts and phosphine ligands, nor expensive starting materials, which provides an industrially applicable, environmentally friendly and economically improved process for obtaining vortioxetine, or salts thereof. The present invention further provides a new intermediate compound that is highly useful as a key intermediate in the preparation of vortioxetine or salts thereof. The intermediate is solid, crystalline, suitable for optional purification by precipitation or preferably recrystallization.

### Detailed Description of the Invention

The invention is described below in further detail by embodiments, without being limited thereto.

A general concept of the process of the present invention may be represented in Scheme 1.

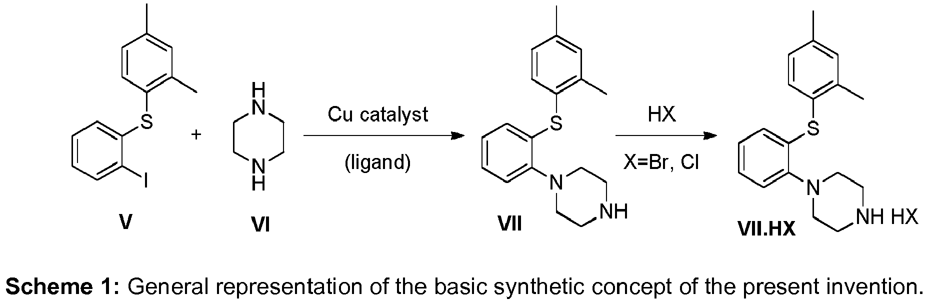

The compound of formula V, i.e. (2,4-dimethylphenyl)(2-iodophenyl)sulfane, is reacted with piperazine (compound of formula **VI)** in the presence of a copper catalyst to form the compound of formula **VII** (vortioxetine). The reaction may be conducted in the presence of an organic or inorganic base, for example, K₂CO₃, Cs₂CO₃, Na₂CO₃, K₃PO₄, NaOtBu, KOtBu, NaOH, KOH, Et₃N, Et₂NiPr, Bu₃N, triethanolamine, DBU, DABCO, or the like, in a polar or non-polar solvent. Suitable solvents are DMSO, DMF, NMP, dioxane, toluene, iPrOH. The temperature of the reaction should be in a range of from 20°C to 200°C, preferably the reaction temperature is about 120°C.

The copper catalyst used is, for example, Cu(I) salt, such as CuI, CuBr, CuCI or Cu₂O. The reaction presented in Scheme 1 can be performed without a ligand. Its presence, however, increases the reaction speed. Most commonly used ligands are amino acids, for example, proline, glycine, sarcosine (N-methylglycine), N,N-dimethylglycine; bypyridines, for example, 2,2'-bipyridyl, 1,10-phenanthroline, neocuproine (2,9-dimethyl[1,10]phenanthroline); diamines, for example, cis- or trans-1,2-diaminocyclohexane, cis- or trans-N,N'-dimethyl-1,2-cyclohexanediamine, 2-methylpropane-1,2-diamine, N,N'-dimethylethylenediamine, N,N,N',N'-tetramethylethylene-diamine; phenols, for example, 2,6-dimethylphenol, 2-phenylphenol, 1,1'-bi(2-naphthol), 8-quinolinol, N,N-diethyl-2-hydroxybenzamide; alcohols, for example, ethane-1,2-diol, 1,1,1-tris-(hydroxylmethyl)ethane; and other types of ligands such as 1H-benzotriazole, 2-((2,6-dimethyl-phenyl)amino)-2-oxoacetic acid, and 2-isobutyrylcyclohexanone. Preferably used ligands are 2-phenylphenol and N,N-diethyl-2-hydroxybenzamide.

Optionally, the vortioxetine compound obtained by the process of the present invention can also be converted to a salt thereof, for example to a hydrobromide salt **(VII.HBr)** or a hydrochloride salt **(VII.HCl).**

In a particular embodiment, the compound of formula **V** may be prepared by a reaction illustrated in Scheme 2.

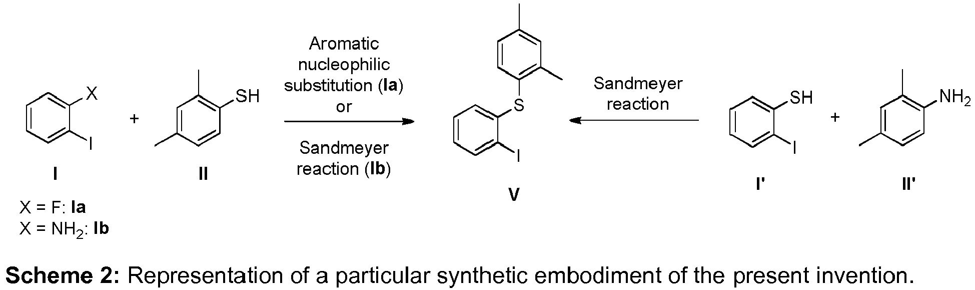

Intermediate **V** can be prepared according to simple nucleophilic aromatic substitution of fluorine atom in compound **Ia** with 2,4-dimethylbenzenethiol **(II).** It can also be prepared according to Sandmeyer reaction conditions starting from 2-iodoaniline **(Ib)** and 2,4-dimethylbenzenethiol **(IIa)** or starting from 2-iodobenzenethiol **(I')** and 2,4-dimethylaniline **(II').**

Alternatively, (2,4-Dimethylphenyl)(2-iodophenyl)sulfane **(V)** can also be prepared according to the reaction path presented in Scheme 3 through 2-((2,4-dimethylphenyl)thio)aniline **(III),** which can be prepared directly from 2-aminobenzenethiol **(I")** coupled with 1-bromo-2,4-dimethylbenzene **(II"a)** or 1-iodo-2,4-dimethylbenzene **(II"b)** using different types of heavy metal catalysts, for example, Pd, Cu, Rh, Co, Fe, Ni, In, La, Zr. Most commonly used heavy metals for such transformations are Pd and Cu.

Compound **III** can also be prepared from 2,4-dimethylbenzenethiol **(II)** and 1-fluoro-2-nitrobenzene **(I"'a)** or 1-chloro-2-nitrobenzene **(I"'b).** In the first step (2,4-dimethylphenyl)(2-nitrophenyl)sulfane **(III')** is formed and in the second reaction step nitro group is reduced to amino.

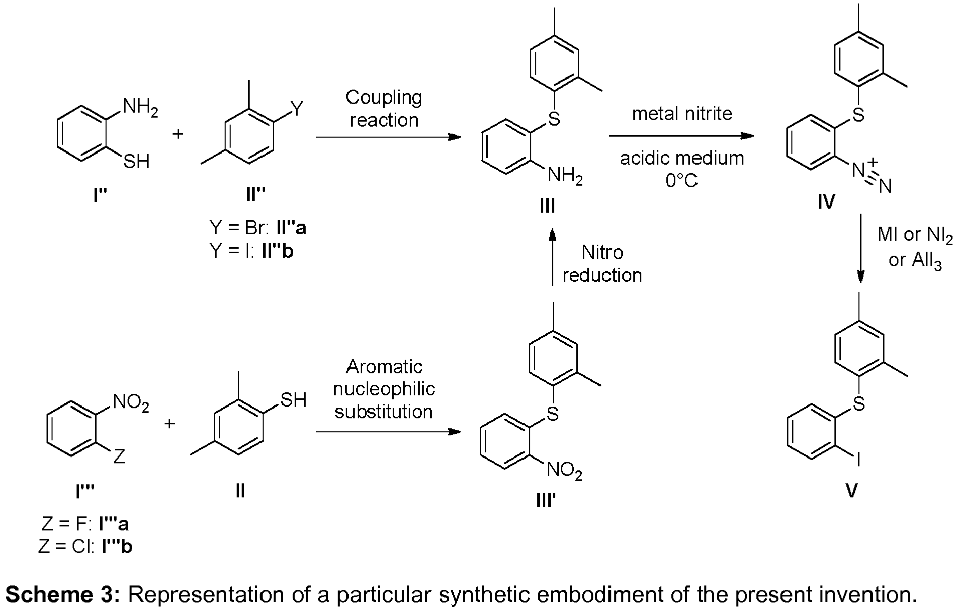

Compound **III** obtained as described above is then transformed to (2,4-dimethylphenyl)(2-iodophenyl)sulfane (V) using Sandmeyer reaction conditions. This can be achieved by adding an acidic medium, for example, H₂SO₄ or HCl, to a solution of **III**, which is followed by addition of an inorganic metal nitrite compound, for example, NaNO₂. The reaction is conducted in water or a mixture of water and polar organic solvent, for example MeCN, at a temperature in a range from 0°C to 10°C. The obtained diazo compound is then reacted with MI, NI₂ or AlI₃, wherein M is selected from the group consisting of K, Na, Li, Cs, Zn, Cu, NH₄, and Bu₄N, and wherein N is selected from the group consisting of Mg, Ca, Ba, Fe, Cu, Ni, and Sn. Finally, the compound of formula **V** is obtained at a temperature of about 25°C.

The processes described above allow for preparation of a crystalline intermediate **V** from cheap starting materials without using toxic palladium catalysts.

In a specific embodiment presented in Scheme 4, the 1-fluoro-2-iodobenzene **(Ia)** reacts with 2,4-dimethylbenzenethiol **(II)** in the presence of a base, preferably K₂CO₃ or Cs₂CO₃, in a polar solvent, preferably DMSO or DMF, at reaction temperatures in the range from 80°C to 200°C, preferably the temperature is about 140°C. This reaction, i.e. the aromatic nucleophilic substitution results in affording the (2,4-dimethylphenyl)(2-iodophenyl)sulfane **(V).**

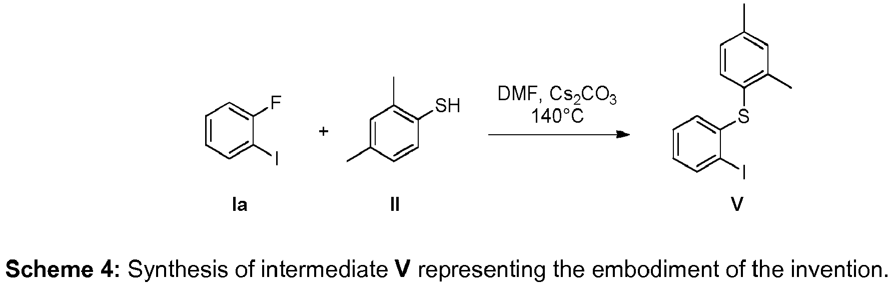

In a further specific embodiment shown in Scheme 5, the compound of formula **V** can be prepared from either 2-iodoaniline **(Ib)** or 2,4-dimethylaniline **(II').** First, the compound **Ib** or **II',** respectively, is dissolved at about 0°C in an acidic media, preferably in the aqueous solution of HCl or H₂SO₄. To the obtained solution NaNO₂ is added. Finally, a water solution of potassium or sodium salt of 2,4-dimethylbenzenethiol **(II)** or 2-iodobenzenethiol **(I')** is then slowly added at 0°C to yield the compound **V.**

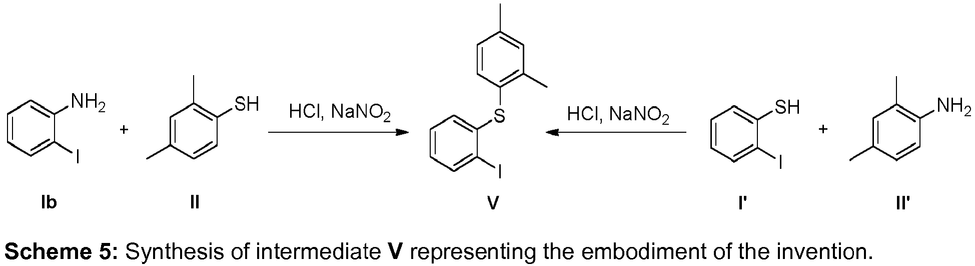

In a further specific embodiment, the compound of formula **V** is prepared by reacting the 2-aminobenzenethiol **(I")** and 1-iodo-2,4-dimethylbenzene **(II"a)** (Scheme 6). These compounds are coupled in a polar solvent, preferably DMSO, using heavy metal catalyst, preferably copper catalyst, for example CuI, in a presence of a base, preferably K₂CO₃, to give intermediate **III.** The preferred reaction temperature is about 120°C. (2,4-Dimethylphenyl)(2-iodophenyl)sulfane **(V)** is then prepared according to standard Sandmeyer reaction conditions from compound **III**, which is first transformed to corresponding diazo compound using diazotizing agent, preferably NaNO₂, in a presence of an acid, preferably HCl or H₂SO₄, at 0°C in water or a mixture of water and appropriate polar organic solvent, preferably MeCN. Finally, an aqueous solution of potassium or sodium iodide is slowly added to the solution/mixture at about 0°C. Compound **V** can be isolated in a crystalline form.

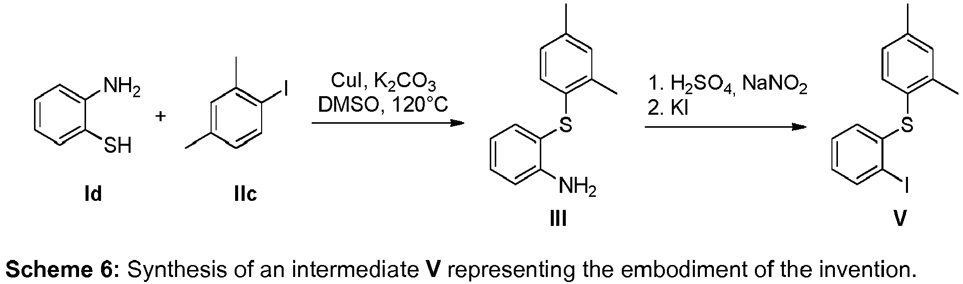

Vortioxetine **(VII)** can be prepared from (2,4-dimethylphenyl)(2-iodophenyl)sulfane **(V)** by heavy metal catalyzed coupling with piperazine **(VI).**

As shown in the reference examples 1 and 2, Pd-catalyzed coupling could be used to obtain vortioxetine from intermediate **V.** In this coupling any palladium(II) source could be used like Pddba₂, Pd₂dba₃, Pd(OAc)₂, PdCl₂, PdBr₂, etc. This transformation occurs in the presence of phosphine (monophosphine or diphosphine) ligands, and also in the presence of carbene-type ligands. For example, Pddba₂/rac-BINAP could be used as a catalytic system.

However, as mentioned above, it is desirable to avoid the use of a palladium catalyst in the synthesis of vortioxetine and the subsequent purification steps required to remove palladium residue from the final pharmaceutical product.

Hence, according to the process of the present invention, the compound of formula **V** is coupled with piperazine **(VI)** in a presence of a copper catalyst, which is made possible due to the presence of a more reactive iodine atom in the compound **V.**

Conversely, and as shown by Comparative Examples 1 and 2, the Cu-catalyzed coupling does not work if (2-bromophenyl)(2,4-dimethylphenyl)sulfane **(V')** is used.

In a specific embodiment presented in Scheme 7, vortioxetine **(VII)** can be prepared by reacting the compound of formula **V** with piperazine **(VI)** in the presence of CuI and a ligand, preferably 2-phenylphenol or N,N-diethyl-2-hydroxybenzamide. The reaction is performed in a polar solvent, preferably in DMSO, in a presence of a base, preferably K₃PO₄, in a temperature range of from 20°C to 200°C, preferably the reaction temperature is about 120°C.

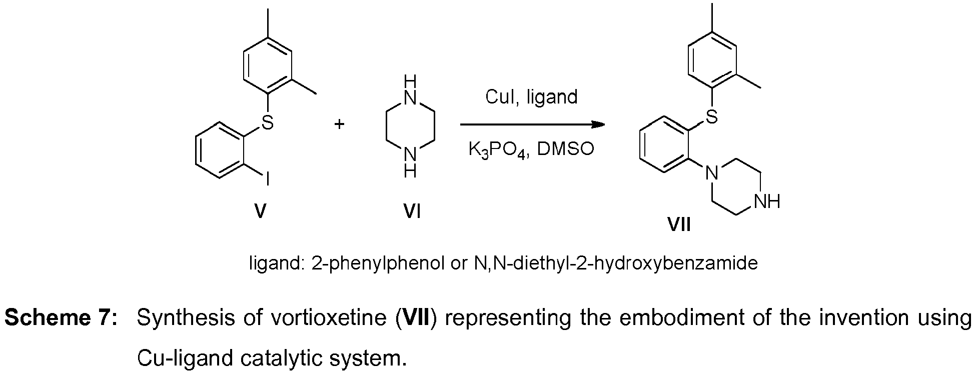

The vortioxetine compound, or a salt thereof, prepared according to the invention may be used or administered on its own, preferably it is administered as a pharmaceutical composition comprising vortioxetine, or a salt thereof, and a pharmaceutically acceptable excipient and/or carrier. Further, the vortioxetine compound, or a salt thereof, prepared according to the invention may be combined with other drugs, especially with drugs used for the treatment of depression and anxiety.

In a further aspect of the present invention, a pharmaceutical composition comprising the compound of formula **VII** (vortioxetine), or a salt thereof, is prepared by comprising the steps of preparing the compound of formula **VII,** or a salt thereof, as described above, and mixing the compound of formula **VII,** or a salt thereof, with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising vortioxetine, or a salt thereof, prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof, or particles containing the same may be coated by another substance. The powder mixture or particles containing the compound may also be dispensed into capsules.

The pharmaceutical composition comprising vortioxetine, or a salt thereof, prepared according to the invention in a desired dose is generally suitable to treat a disease or condition of a patient in need thereof, speciffically to display a desired activity when treating the depression and anxiety.

Further embodiment of the present invention resides in the provision of a valuable intermediate compound V useful in the synthesis of a compound of formula **VII** (vortioxetine)

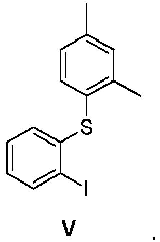

The following Examples are non-limiting and serve to illustrate the invention.

### Experimental Procedures

### Example 1: Preparation of 2-((2,4-dimethylphenyl)thio)aniline (III) from 1-fluoro-2-nitrobenzene (I"'a) or 1-chloro-2-nitrobenzene (I"'b) and 2,4-dimethylbenzenethiol (II) followed by nitro reduction.

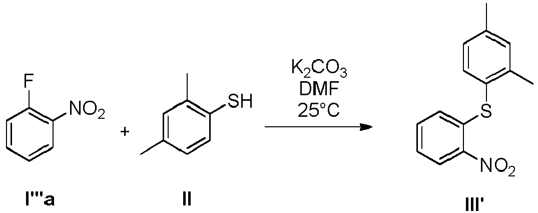

To a the mixture of K₂CO₃ (125 g, 0.90 mol) in dry DMF (0.5 L) while stirring at 25°C was slowly added 2,4-dimethylbenzenethiol **II** (113 mL, 0.84 mol), then 1-fluoro-2-nitrobenzene **I"'a** (116 g, 0,82 mol) was added in 2 h. After stirring the reaction mixture for 30 min 1 L water was added, and obtained mixture was stirred at 25°C for 1 h. Yellow precipitate was then filtered off, washed with water (2 x 0.5 L) and dried in vacuum at 50°C to afford title compound **III'** as yellow crystals (209 g, 98% yield): DSC: Onset 93.34°C, Peak 97.39°C; ¹H NMR (CDCl₃, 500 MHz) *δ* 2.30 (s, 3H), 2.40 (s, 3H), 6.71 (dd, J = 1.2, 8.2 Hz, 1H), 7.11 (m, 1 H), 7.18-7.22 (m, 2H), 7.32 (m, 1 H), 7.46 (d, J = 7.8 Hz, 1 H), 8.25 (dd, J = 1.4, 8.2 Hz, 1 H); MS (ESI) m/z: 260 [MH]⁺.

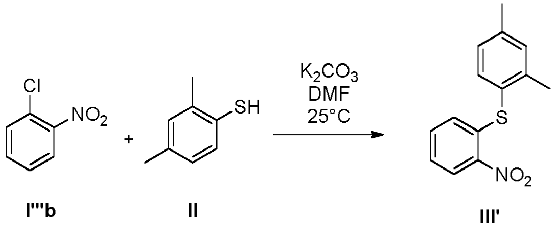

To a solution of 1-chloro-2-nitrobenzene **I"'b** (5.00 g, 31.7 mmol) in dry DMF (30 mL) K₂CO₃ (5.26 g, 38 mmol) and 2,4-dimethylbenzenethiol **II** (4.50 mL, 33.3 mmol) were added, and resulting reaction mixture was stirred at 25°C for 18 h. Water (60 mL) was added and obtained mixture was stirred at 25°C for 30 min. Yellow precipitate was then filtered off, washed with water (2 x 50 mL) and dried to afford title compound **III'** as yellow crystals (8.24 g, total yield): ¹H NMR (CDCl₃, 500 MHz) *δ* 2.30 (s, 3H), 2.40 (s, 3H), 6.71 (dd, *J* = 1.2, 8.2 Hz, 1 H), 7.11 (m, 1 H), 7.18-7.22 (m, 2H), 7.32 (m, 1H), 7.46 (d, *J* = 7.8 Hz, 1 H), 8.25 (dd, *J* = 1.4, 8.2 Hz, 1 H); MS (ESI) m/z: 260 [MH]⁺.

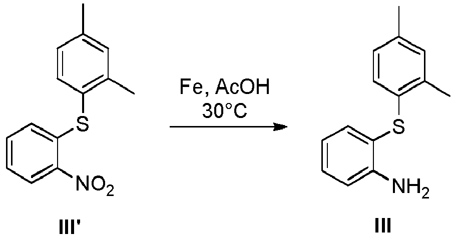

To a mixture of (2,4-dimethylphenyl)(2-nitrophenyl)sulfane **III'** (10.0 g, 38.6 mmol) and AcOH (100 mL) Fe (8.61 g, 154 mmol) was added, and resulting reaction mixture was stirred at 30°C for 16 h. Reaction mixture was then filtered through a bed of Celite, and filtrate was concentrated. To the residue 300 mL saturated NaHCO₃ and 100 mL EtOAc was added. Organic layer was separated, water layer was washed with EtOAc (100 mL), combined organic layers were dried over Na₂SO₄, and solvent was evaporated to afford title compound **III** as orange oil (8.85 g, 99% yield): ¹H NMR (CDCl₃, 500 MHz) *δ* 2.28 (s, 3H), 2.40 (s, 3H), 4.24 (br s, 2H), 6.71 (d, *J* = 8.1 Hz, 1 H), 6.75-6.81 (m, 2H), 6.87 (m, 1 H), 7.02 (m, 1 H), 7.23 (m, 1 H), 7.38 (dd, *J* = 1.5, 7.7 Hz, 1 H); MS (ESI) *m*/*z*: 230 [MH]⁺.

### Example 2: Preparation of 2-((2,4-dimethylphenyl)thio)aniline (III) from 2-aminobenzenethiol (I")

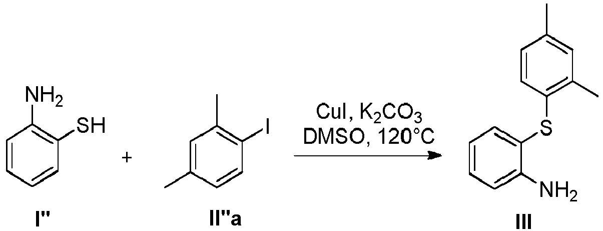

Mixture of 2-aminobenzenethiol **I**" (1.07 mL, 10.0 mmol), 1-iodo-2,4-dimethylbenzene **II"a** (1.43 mL, 10.0 mmol), CuI (0.19 g, 1.0 mmol), K₂CO₃ (6.90 g, 50.0 mmol), and dry and degasses DMSO (50 mL) was stirred under nitrogen at 120°C for 16 h. Reaction mixture was cooled to room temperature, water (50 mL) was added and the product was extracted to EtOAc (2 x 150 mL). Combined organic layers were washed with water (3 x 150 mL) and brine (3 x 150 mL). Organic phase was then dried over MgSO₄ and solvent was evaporated to afford title compound as a dark oil (2.15 g, 94% yield): ¹H NMR (CDCl₃, 500 MHz) *δ* 2.28 (s, 3H), 2.40 (s, 3H), 4.24 (br s, 2H), 6.71 (d, J = 8.1 Hz, 1 H), 6.75-6.81 (m, 2H), 6.87 (m, 1 H), 7.02 (m, 1 H), 7.23 (m, 1 H), 7.38 (dd, J = 1.5, 7.7 Hz, 1 H); MS (ESI) m/z: 230 [MH]⁺.

### Example 3: Preparation of (2,4-dimethylphenyl)(2-iodophenyl)sulfane (V) from 2-((2,4-dimethylphenyl)thio)aniline (III)

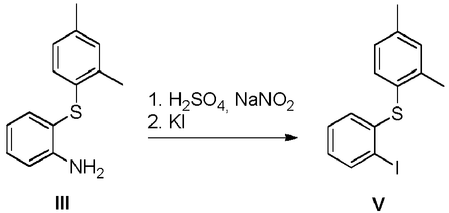

To a mixture of 2-((2,4-dimethylphenyl)thio)aniline **III** (1.83 g, 8.0 mmol) in water (8 mL) slowly H₂SO₄ (96%, 1.2 mL) was added. Obtained mixture was cooled to 0°C and solution of NaNO₂ (0.61 g, 8.8 mmol) in water (2 mL) was added slowly. After addition was complete MeCN (5 mL) was added and obtained mixture was stirred at 0°C for 30 min. This solution was then slowly added to an ice-cold aqueous (4 mL water) solution of KI (1.74 g, 10.5 mmol). Obtained mixture was stirred at 25°C for 16 h. EtOAc (20 mL) was then added, organic layer was separated, washed with water (3 x 50 mL) and Na₂S₂O₅ (aq., 25 mL), and dried over MgSO₄ containing C (1 g) and silicagel (1 g). Salts were then filtered off, washed with EtOAc, and filtrate was concentrated to afford title compound **V** as an orange oil which crystalized upon standing (2.47 g, 91% yield): ¹H NMR (CDCl₃, 500 MHz) *δ* 2.33 (s, 3H), 2.37 (s, 3H), 6.58 (dd, *J* = 1.5, 8.0 Hz, 1 H), 6.81 (m, 1 H), 7.06 (m, 1 H), 7.13 (m, 1 H), 7.17 (m, 1 H), 7.39 (d, *J* = 7.8 Hz, 1 H), 7.80 (dd, *J* = 1.3, 7.8 Hz, 1 H); MS (ESI) *m*/*z*: 341 [MH]⁺.

### Example 4: Preparation of (2,4-dimethylphenyl)(2-iodophenyl)sulfane (III) from 1-fluoro-2-iodobenzene (Ia)

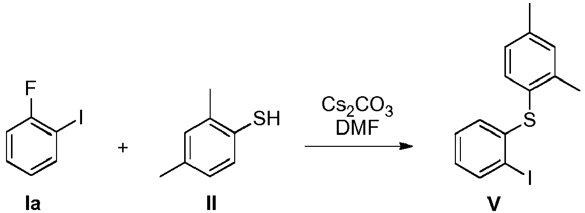

Mixture of 1-fluoro-2-iodobenzene **Ia** (0.58 mL, 5.0 mmol), 2,4-dimethylbenzenethiol **II** (0.68 mL, 5.0 mmol) and Cs₂CO₃ (1.71 g, 5.25 mmol) in dry and degasses DMF (2 mL) was stirred under nitrogen at 140°C for 3 days. Reaction mixture was cooled to room temperature, water (25 mL) was then added and the product was extracted to EtOAc (2 x 20 mL). Combined organic layers were washed with brine (2 x 20 mL) and dried over MgSO₄. After evaporation of the solvent title compound **V** was isolated as brown oil (1.29 g, 75% yield): ¹H NMR (CDCl₃, 500 MHz) *δ* 2.33 (s, 3H), 2.37 (s, 3H), 6.58 (dd, J = 1.5, 8.0 Hz, 1 H), 6.81 (m, 1 H), 7.06 (m, 1 H), 7.13 (m, 1 H), 7.17 (m, 1 H), 7.39 (d, J = 7.8 Hz, 1 H), 7.80 (dd, J = 1.3, 7.8 Hz, 1 H); MS (ESI) m/z: 341 [MH]⁺.

### Example 5: Preparation of (2,4-dimethylphenyl)(2-iodophenyl)sulfane (III) from 2-iodoaniline (Ib)

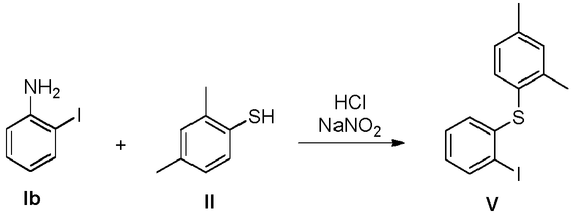

To a mixture of ice (2.5 g) and 37% HCl (2.1 mL) 2-iodoaniline **Ib** (2.74 g, 12.5 mmol) is slowly added. After obtained mixture was stirred at 0°C for 15 min, aqueous (2.15 mL water) solution of NaNO₂ (0.88 g, 12.75 mmol) was slowly added. While obtained solution was stirred at 0°C for 30 min solution of KOH (2.8 g, 50.0 mmol), 2,4-dimethylbenzenethiol **II** (2.03 mL, 15.0 mmol) in water (5 mL) was prepared. To this solution previously prepared solution with diazonium salt was slowly added. Obtained reaction mixture was stirred at 5°C for 1 h, then it was heated to 80°C and stirred for 20 h. Reaction mixture was then cooled to room temperature, water (20 mL) was added and the product was extracted to MeTHF (2 x 20 mL). Combined organic layers were washed with 1 M HCl (3 x 20 mL) and brine (2x 20 mL). Organic phase was separated, dried over MgSO₄ and solvent was evaporated. Obtained crude product was purified by chromatography (MeCy) to afford title compound as orange oil (2.35 g, 55% yield): ¹H NMR (CDCl₃, 500 MHz) *δ* 2.33 (s, 3H), 2.37 (s, 3H), 6.58 (dd, *J* = 1.5, 8.0 Hz, 1 H), 6.81 (m, 1 H), 7.06 (m, 1 H), 7.13 (m, 1 H), 7.17 (m, 1 H), 7.39 (d, *J* = 7.8 Hz, 1 H), 7.80 (dd, *J* = 1.3, 7.8 Hz, 1 H); MS (ESI) m/z: 341 [MH]⁺.

### Example 6: Preparation of (2,4-dimethylphenyl)(2-iodophenyl)sulfane (V) from 2-iodobenzenethiol (I')

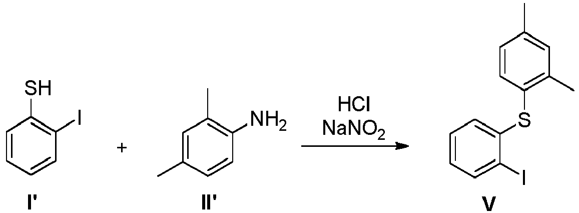

To a mixture of H₂SO₄ (96%, 2.0 mL) and water (10 mL) 2,4-dimethylaniline **II'** (1.54 mL, 12.5 mmol) was slowly added. After cooling to 0°C, NaNO₂ (0.88 g, 12.75 mmol) was slowly added. After stirring at 0°C for 30 min, it was slowly added to an ice-cold solution of 2-iodobenzenethiol **(I')** (3.0 g, 12.75 mmol) and KOH (5.6 g, 100 mmol) in water (15 mL). Obtained reaction mixture was stirred at 5°C for 1 h, then MeCN (20 mL) was added, solution was heated to 70°C and stirred for 16 h. Water (50 mL) was then added, the product was extracted to EtOAc (3 x 25 mL), combined organic layers were dried over MgSO₄. After evaporation of the solvent crude product was purified by chromatography (MeCy) to afford title compound as orange oil which crystalized upon standing (2.60 g, 61% yield): ¹H NMR (CDCl₃, 500 MHz) *δ* 2.33 (s, 3H), 2.37 (s, 3H), 6.58 (dd, J = 1.5, 8.0 Hz, 1 H), 6.81 (m, 1 H), 7.06 (m, 1 H), 7.13 (m, 1 H), 7.17 (m, 1 H), 7.39 (d, J = 7.8 Hz, 1 H), 7.80 (dd, J = 1.3, 7.8 Hz, 1 H); MS (ESI) m/z: 341 [MH]⁺.

### Example 7: Preparation of 1-(2-((2,4-dimethylphenyl)thio)phenyl)piperazine (vortioxetine, VII)

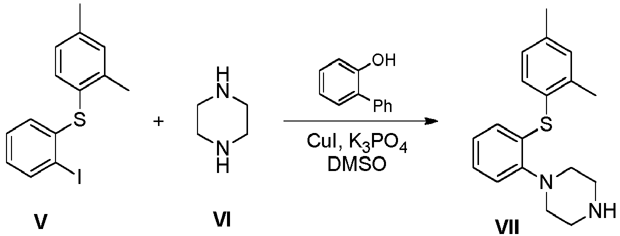

Mixture of (2,4-dimethylphenyl)(2-iodophenyl)sulfane V (0.34 g, 1.0 mmol), piperazine **VI** (0.13 g, 1.5 mmol), K₃PO₃ (0.42 g, 2.0 mmol), CuI (19 mg, 0.1 mmol), and 2-phenylphenol (68 mg, 0.4 mmol) in dry and degassed DMSO (2 mL) was heated under nitrogen atmosphere at 120°C for 20 h. Water (10 mL) is then added and product is extracted to EtOAc (3 x 10 mL). Combined organic layers were washed with water (3 x 10 mL) and brine (2 x 10 mL) and dried over Na₂SO₄. After evaporation of the solvent crude product is purified by chromatography to afford title compound: ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1 H), 6.87 (m, 1 H), 7.04 (m, 1 H), 7.06-7.10 (m, 2H), 7.16 (m, 1 H), 7.39 (d, *J* = 7.8 Hz, 1 H); MS (ESI) m/z: 299 [MH]⁺.

### Example 8: Preparation of 1-(2-((2,4-dimethylphenyl)thio)phenyl)piperazine (vortioxetine, VII)

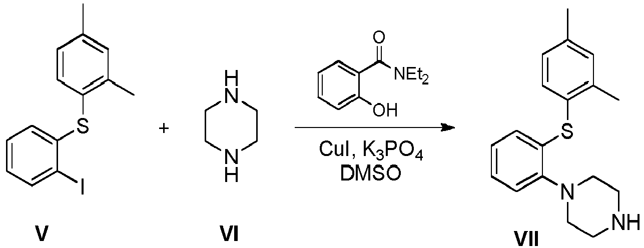

Mixture of (2,4-dimethylphenyl)(2-iodophenyl)sulfane **V** (0.34 g, 1.0 mmol), piperazine **VI** (0.13 g, 1.5 mmol), K₃PO₃ (0.42 g, 2.0 mmol), CuI (19 mg, 0.1 mmol), and N,N-diethyl-2-hydroxybenzamide (39 mg, 0.2 mmol) in dry and degassed DMSO (2 mL) was heated under nitrogen atmosphere at 120°C for 20 h. Water (10 mL) is then added and product is extracted to EtOAc (3 x 10 mL). Combined organic layers were washed with water (3 x 10 mL) and brine (2 x 10 mL) and dried over Na₂SO₄. After evaporation of the solvent crude product is purified by chromatography to afford title compound: ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1 H), 6.87 (m, 1 H), 7.04 (m, 1 H), 7.06-7.10 (m, 2H), 7.16 (m, 1 H), 7.39 (d, J = 7.8 Hz, 1 H); MS (ESI) m/z: 299 [MH]⁺.

### Example 9: Preparation of 1-(2-((2,4-dimethylphenyl)thio)phenyl)piperazine hydrobromide (vortioxetine HBr, VII.HBr)

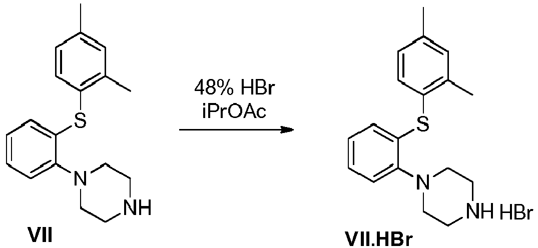

To a solution of vortioxetine **VII** (1.80 g, 6.03 mmol) in iPrOAc (20 mL) at room temperature 48% HBr (0.68 mL, 6.03 mmol) was slowly added. Obtained mixture was stirred at room temperature for 1 h, white precipitate was then filtered off, washed with acetone (2 x 20 mL), and dried to afford title compound **VII.HBr** as a white powder (2.15 g, 94% yield): ¹H NMR (DMSO-*d*₆, 500 MHz) *δ* 2.23 (s, 3H), 2.32 (s, 3H), 3.15-3.27 (m, 8H), 6.40 (m, 1 H), 6.96 (m, 1 H), 7.08-7.17 (m, 3H), 7.24 (m, 1 H), 7.32 (d, *J* = 7.8 Hz, 1 H), 8.85 (br, 2H).

### Reference Example 1: Preparation of 1-(2-((2,4-dimethylphenyl)thio)phenyl)piperazine (vortioxetine, VII)

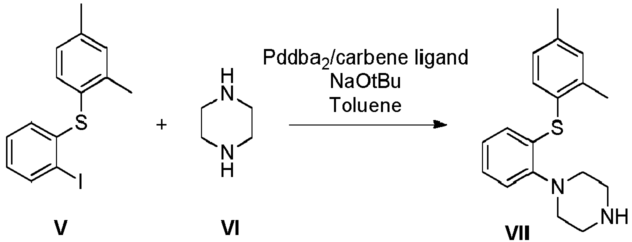

Mixture of piperazine (1.0 g, 11.6 mmol), NaOtBu (1.37 g, 13.8 mmol), Pddba₂ (40 mg, 0.07 mmol), and 1,3-bis(2,6-di-i-propylphenyl)imidazolium chloride (24 mg, 0,07 mmol) in dry and degassed toluene (10 mL) is stirred at room temperature for 1 h. (2,4-Dimethylphenyl)(2-iodophenyl)sulfane **V** (1.32 g, 3.86 mmol) is then added, reaction mixture is heated to 100°C and stirred for 24 h. After cooling to room temperature to the reaction mixture water (5 mL) and Celite (0.4 g) is added. After stirring for 20 min salts are filtered off, organic layer is separated, washed with brine (2 x 10 mL), dried over Na₂SO₄ and solvent is evaporated to afford crude product, which is then purified by chromatography to afford title compound as yellowish crystals: ¹H NMR (CDCl₃, 500 MHz) *δ* 1.63 (br s, 1 H), 2.33 (s, 3H), 2.37 (s, 3H),

### Reference Example 2: Preparation of 1-(2-((2,4-dimethylphenyl)thio)phenyl)piperazine (vortioxetine, VII)

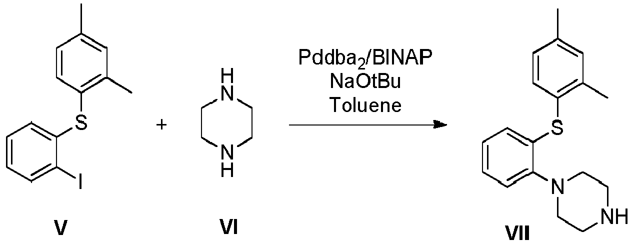

Mixture of piperazine (1.29 g, 15.0 mmol), NaOtBu (1.77 g, 17.8 mmol), Pddba2 (52 mg, 0.09 mmol), and rac-BINAP (93 mg, 0,15 mmol) in dry and degassed toluene (10 mL) was stirred at room temperature for 1 h. (2,4-Dimethylphenyl)(2-iodophenyl)sulfane **V** (1.70 g, 5.0 mmol) was then added, reaction mixture was heated to 100°C and stirred for 24 h. After cooled to room temperature to the reaction mixture water (5 mL) and Celite (0.4 g) were added. After stirring for 20 min salts were filtered off, organic layer was separated, washed with brine (2 x 10 mL), dried over Na₂SO₄ and solvent was evaporated to afford product as an orange oil (1.41 g, 95% yield): ¹H NMR (CDCl₃, 500 MHz) *δ* 1.63 (br s, 1 H), 2.33 (s, 3H), 2.37 (s, 3H), 3.02-3.09 (m, 8H), 6.52 (m, 1H), 6.87 (m, 1H), 7.04 (m, 1 H), 7.06-7.10 (m, 2H), 7.16 (m, 1 H), 7.39 (d, *J* = 7.8 Hz, 1 H); MS (ESI) *m*/*z*: 299 [MH]⁺.

### Comparative Example 1: Preparation of 1-(2-((2,4-dimethylphenyl)thio)phenyl)piperazine (vortioxetine, VII)

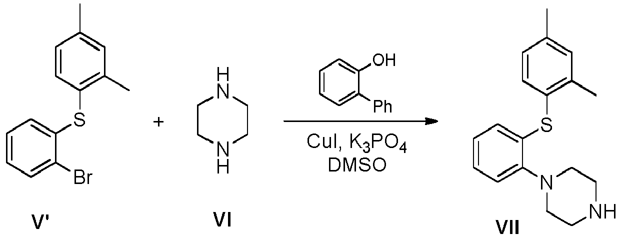

Mixture of (2,4-dimethylphenyl)(2-bromohenyl)sulfane V' (0.29 g, 1.0 mmol), piperazine **VI** (0.13 g, 1.5 mmol), K₃PO₃ (0.42 g, 2.0 mmol), CuI (19 mg, 0.1 mmol), and 2-phenylphenol (68 mg, 0.4 mmol) in dry and degassed DMSO (2 mL) was heated under nitrogen atmosphere at 120°C for 20 h. Vortioxetine **VII** was not formed.

### Comparative Example 2: Preparation of 1-(2-((2,4-dimethylphenyl)thio)phenyl)piperazine (vortioxetine, VII)

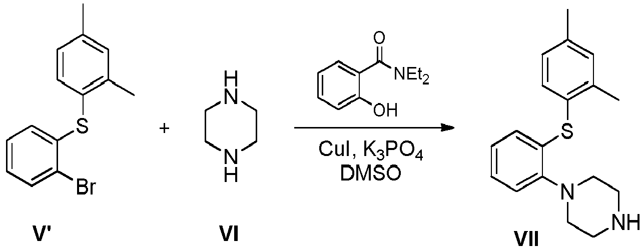

Mixture of (2,4-dimethylphenyl)(2-bromophenyl)sulfane V' (0.29 g, 1.0 mmol), piperazine **VI** (0.13 g, 1.5 mmol), K₃PO₃ (0.42 g, 2.0 mmol), CuI (19 mg, 0.1 mmol), and N,N-diethyl-2-hydroxybenzamide (39 mg, 0.2 mmol) in dry and degassed DMSO (2 mL) was heated under nitrogen atmosphere at 120°C for 20 h. Vortioxetine **VII** was not formed.

## Claims

1. A process for the preparation of a compound of formula **VII,** or a salt thereof, 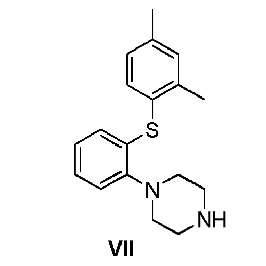 said process comprising the steps of
a) reacting a compound of formula V 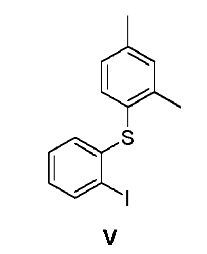 with piperazine in a presence of a copper catalyst, and
b) optionally, converting the obtained compound of formula **VII** into the salt thereof.

2. The process according to claim 1, wherein a Cu(I) salt is used as the copper catalyst.

3. The process according to claim 2, wherein the Cu(I) salt is selected from the group consisting of CuI, CuBr, CuCI and Cu₂O.

4. The process according to claim 2 or claim 3, wherein the copper catalyst additionally comprises a ligand.

5. The process according to claim 4, wherein the ligand is selected from the group consisting of amino acids, bipyridines, diamines, phenols, alcohols, 1H-benzotriazole, 2-((2,6-dimethylphenyl)amino)-2-oxoacetic acid, and 2-isobutyrylcyclohexanone.

6. The process according to claim 5, wherein the ligand is 2-phenylphenol or N,N-diethyl-2-hydroxybenzamide.

7. The process according to claim 1, wherein the compound of formula **V** is prepared by reacting a compound of formula **I** 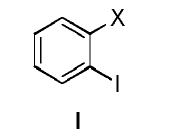 wherein X represents F or NH₂, with a compound of formula **II**

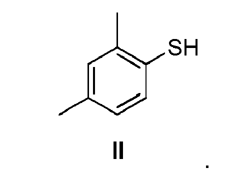

8. The process according to claim 1, wherein the compound of formula V is prepared by reacting a compound of formula I' 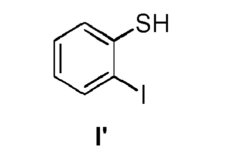 with a compound of formula **II'**

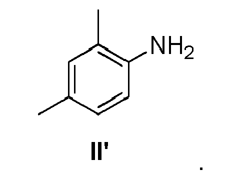

9. The process according to claim 1, wherein the compound of formula V is prepared by
reacting a compound of formula **III** 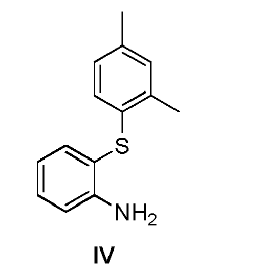 with nitrite, and
reacting the obtained diazo compound of formula **IV'** 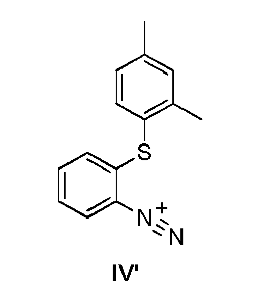 with a compound of formula **MI,** wherein M is selected from the group consisting of K, Na, Li, Cs, Zn, Cu, NH₄, and Bu₄N, or with a compound of formula **NI₂,** wherein N is selected from the group consisting of Mg, Ca, Ba, Fe, Cu, Ni, and Sn, or with AlI₃.

10. The process according to claim 9, wherein the compound of formula **III** is prepared by reacting a compound of formula **I"** 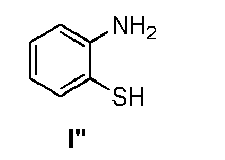 with a compound of formula **II"** 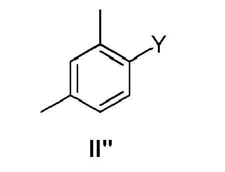 wherein Y represents Br or I.

11. The process according to claim 9, wherein the compound of formula **III** is prepared by
c) reacting a compound of formula **I"'** 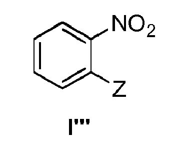 wherein Z represents F or Cl, with a compound of formula **II** 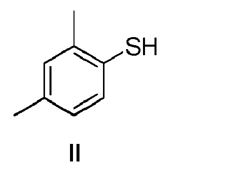 to obtain the compound of formula **III'** 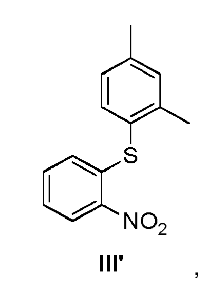 and
d) reducing the compound of formula **III'** to obtain the compound of formula **III.**

12. The process according to any one of the previous claims, wherein the salt of the compound of formula **VII** is a hydrobromide salt or a hydrochloride salt.

13. A compound of formula **V**

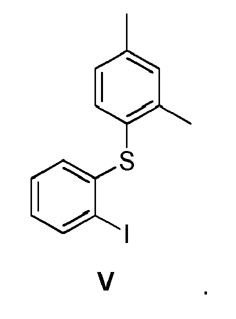

14. Use of the compound as defined in claim 13 in the preparation of vortioxetine or a salt thereof.

15. A process for the preparation of a pharmaceutical composition comprising a compound of
formula **VII,** or a salt thereof 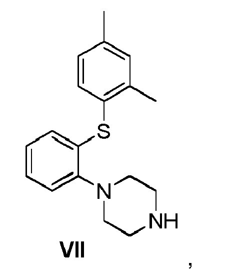 comprising the steps of:
x) preparing a compound of formula **VII,** or a salt thereof, by carrying out a process according to any one of claims 1 to 12, and
y) mixing the compound of formula **VII,** or a salt thereof, with a pharmaceutically acceptable carrier and/or excipient.
